## Europäisches Patentamt
## European Patent Office
## Office européen des brevets

(19)

(11) Publication number: **0 076 964**
**B1**

(12) **EUROPEAN PATENT SPECIFICATION**

(45) Date of publication of patent specification: **28.08.85**

(21) Application number: **82108947.1**

(22) Date of filing: **28.09.82**

(51) Int. Cl.⁴: **A 23 K 1/12,** A 23 K 1/00

(54) **Livestock feed of high nutrient value, and the process and apparatus for its production from waste vegetable materials.**

(30) Priority: **13.10.81 IT 2447281**

(43) Date of publication of application:
**20.04.83 Bulletin 83/16**

(45) Publication of the grant of the patent:
**28.08.85 Bulletin 85/35**

(84) Designated Contracting States:
**AT BE CH DE FR GB LI LU NL SE**

(56) References cited:
**DE-A-2 529 997**
**DE-A-2 849 341**

(73) Proprietor: **ALFA STRAW INTERNATIONAL S.r.l.**
**Vicolo Parentino, 12**
**Padova (IT)**

(72) Inventor: **Rossi, Carlo**
**Via Trento e Trieste, 11**
**Motta di Livenza Treviso (IT)**
Inventor: **Drago, Domenico**
**Via Asiago, 26**
**Schio Vicenza (IT)**

(74) Representative: **Gervasi, Gemma et al**
**Studio Brevetti e Marchi NOTARBARTOLO &**
**GERVASI 33, Viale Bianca Maria**
**I-20122 Milano (IT)**

EP 0 076 964 B1

Courier Press, Leamington Spa, England.

**Description**

This invention relates to a new livestock feed of high nutrient value, and the process and apparatus for its production from waste vegetable materials. More precisely, the invention relates to a new livestock feed obtained from straw, maize stalks and other vegetable products of high cellulose and lignin content which are unusable or usable only to a small extent in livestock feeding, by means of a new guided and controlled physical-chemical-biochemical process which induces fermentation which is initially microaerobic and then takes place under strictly anaerobiosis conditions, these latter being ensured by the blanket of $CO_2$ which forms above the mass as the fermentation gets underway. Whereas according to known processes (see for example German Patent 2.529.997) the waste vegetable materials having high content of cellulose and lignin are treated chemically (alkalization), the new process induces and causes maximum activation of the prevalently cellulolytic endogenous flora, this flora being perfectly compatible with ruminant animal flora and therefore able to subsequently act as a booster therefor. In addition the new process leads to natural enrichment in substances which are highly useful for animals, at the expense of fibrous materials of low natural digestibility. The invention also relates to a new apparatus specifically suitable for production of the new feed. Cattle fodder is known to be generally derived from specific crops, of which the entire above-ground part of the plants and in certain cases also their below-ground part are administered to the animals in the fresh or preserved state. Because of the continuous development of livestock breeding, those areas intended for fodder crops are highly inadequate and difficult to cultivate to a level which satisfies the increasing need.

There are also a great many crops intended for human feeding, such as wheat, maize, barley and the like, which after harvesting the useful part leave large masses of vegetable residue which in practice cannot be used under high conversion conditions for livestock feeding and/or which are partly disposed of by burning.

On this basis, it is easy to imagine the great interest in a process which enables livestock feeding requirements to be satisfied, without using new areas to be subtracted from human feeding.

Reference will be made hereinafter for simplicity to the processing of straw, but this term is intended to also include vegetable residues deriving from cereals, any other fibrous material of vegetable origin either alone or in mixture, maize stalks and the like.

The process according to the present invention comprises the following essential stages:

1—Preparing the straw into pieces having a length not exceeding 7 mm. This mechanical treatment enables the cellulose and lignin macromolecules to be split, so facilitating their successive chemical attack. In addition it leads to a large degree of rupture of the cell wall, with consequent release of the endocellular content. The endocellular content represents the initial medium for triggering the fermentation.

2—Impregnating the finely ground straw with a solution having the following composition for 100 kg of processed straw:

| | |
|---|---|
| a) water | 200—300 litres |
| b) $KMnO_4$ | 0.25—0.35 g |
| c) $FeSO_4 . 7H_2O$ | 40—60 g |
| d) $CuSO_4 . 5H_2O$ | 8—12 g |
| e) dicalcium phosphate | 250—500 g |
| f) molasses | 1—5 kg |
| g) urea | 1.5—3.5 kg |

The overall purpose of said solution is to trigger and feed a straw fermentation process, which induces natural selection of the microbic flora normally present in said straw, mainly favouring the cellulolytic fraction and all those fermentation processes which lead to nutrient products such as proteins, amino acids, carbohydrates, fatty acids and the like.

Each of the components of the aforesaid solution has a specific function in the process.

Specifically, it has been found that the inorganic salts (b), (c), (d) intervene in the selection of the microbic flora to catalyse and regulate the fermentation processes which it is desired to induce and facilitate. A deficiency or excess of one or more of the said mineral salts makes the progress of the fermentation uncontrollable, in that it favours microorganism strains which are different from those useful for the purposes of the invention.

If the process according to the present invention is proceeding normally, then the temperature curve of the processed mass should be bell-shaped up to 55°—65°C. When the temperature of the mass again falls to about 40°C, the process is to be considered terminated.

2

The purpose of the water is to soak and swell the pectins, and weaken the physical cellulose, hemicellulose and lignin bonds, so facilitating their subsequent lysis.

In addition, the water makes available in the biomass the cellular content which emerges from the cells broken down by the mechanical straw trituration treatment, which is indispensable to the selective microflora process.

The urea (g) releases ammonia, which has a double purpose: initially, its action is chemical in splitting the lignin-hemicellulolytic bonds; later, it supplies the nitrogen necessary for the development of the cellulolytic microflora and the fermentation processes, with the formation of nitrogenated compounds which are highly useful for livestock feeding.

The purpose of the molasses (f) is to supply immediately available energy which is necessary for triggering the fermentation.

The fermentation is subsequently sustained by the sugars which originate from the cellulose and hemicellulose lysis.

The purpose of the dicalcium phosphate is to buffer the solution against the ammonia, and represents a feed supplement which remains as such in the final fodder mass.

3—Fermentation

In general, the product can be used between the 10th and 25th day of fermentation, but preferably after 12—20 days.

The table (Table 1) given hereinafter shows the most significant components of the product expressed as a percentage by weight of dry substance on the 12th and 20th day from the beginning of the fermentation process.

The final product normally contains 70—75% of moisture.

In reality, the product can be used after only 48 hours of fermentation if warm water at 50°C is used, if the urea is used in its minimum quantity, and if the phosphates are used in their maximum quantity.

From the given data it is apparent that a cellulose mass which up to the present time could hardly be used as an animal feed has been converted into a mass of high nutrient value by means of a "guided" fermentation process using induced selection of the microbic flora normally present in the processed cellulose mass.

TABLE 1

| Mean values referred to the dry substance | 12 days | 20 days |
|---|---|---|
| Total nitrogenated substances % | 7—11 | 7—11 |
| Lipids % | 0.4—0.9 | 0.6—1 |
| Fibre % | 33—39 | 30—36 |
| Ash % | 5—10 | 6—10 |
| Extractable non-nitrogenated % | 54.6—39.1 | 57.4—42.0 |
| Metabolisable energy Kcal | 2300 | 2300 |

| Other characteristic values | $\bar{x}$ | s. | c.v. | $\bar{x}$ | s. | c.v. |
|---|---|---|---|---|---|---|
| Acetic acid % | 4.50 | 0.2973 | 6.60 | 4.72 | 0.6502 | 13.77 |
| Butyric acid % | 0.75 | 0.2124 | 28.00 | 1.12 | 1.0400 | 92.82 |
| Propionic acid % | 0.35 | 0.1003 | 28.65 | 0.49 | 0.2831 | 57.70 |
| Volatile fatty acids % | 5.60 | — | — | 6.33 | — | — |
| Lactic acid % | 0.80 | 0.4975 | 62.18 | 0.52 | 0.4753 | 91.40 |
| Total fatty acids % | 6.40 | — | — | 6.85 | — | — |
| pH | 7.0 | 0.8081 | 11.45 | 7.41 | 0.6852 | 9.24 |

$\bar{x}$=mean
s.=standard deviation
c.v.=variation coefficient.

A particularly interesting aspect of the present invention is the possibility of using spent sugarbeet pulp, distillery wash, rotted apples and grape skins, with their entire moisture content as a partial or total replacement for the processed water.

This alternative of the new process goes some way to solving the large problems inherent in spent sugarbeet pulp, by avoiding the high energy consumption required to dry it, and the heavy investment required by purification plants.

The use of spent sugarbeet pulp and the like in place of water means that the mixing must obviously be carried out by suitable apparatus.

As initially stated, a second subject matter of the present invention is the apparatus for producing the new fodder mass.

The overall structure of this apparatus, its component parts and its operation will be more apparent with reference to the accompanying drawings. In the various drawings, the same parts are indicated by the same reference numerals.

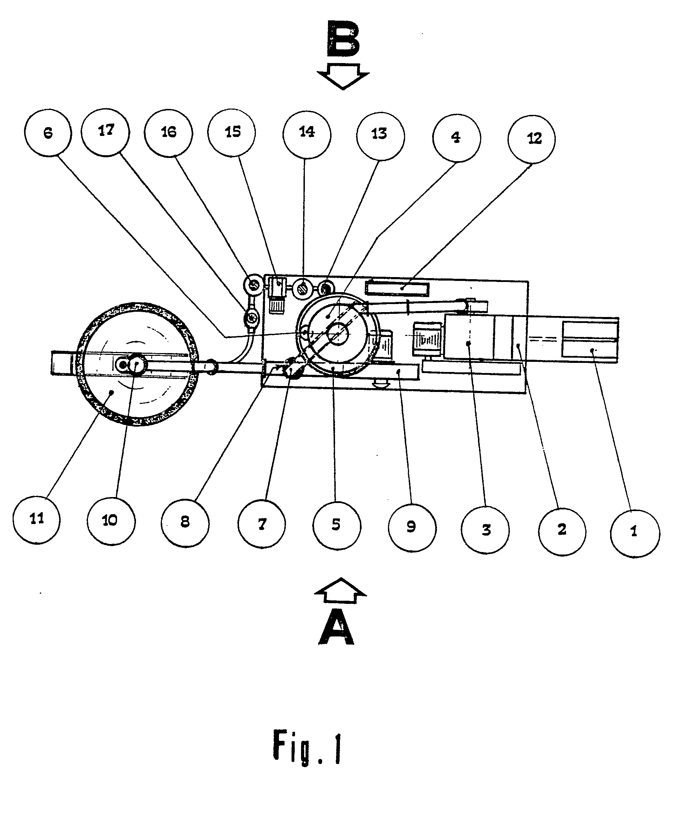

Figure 1 is a plan view of the apparatus.

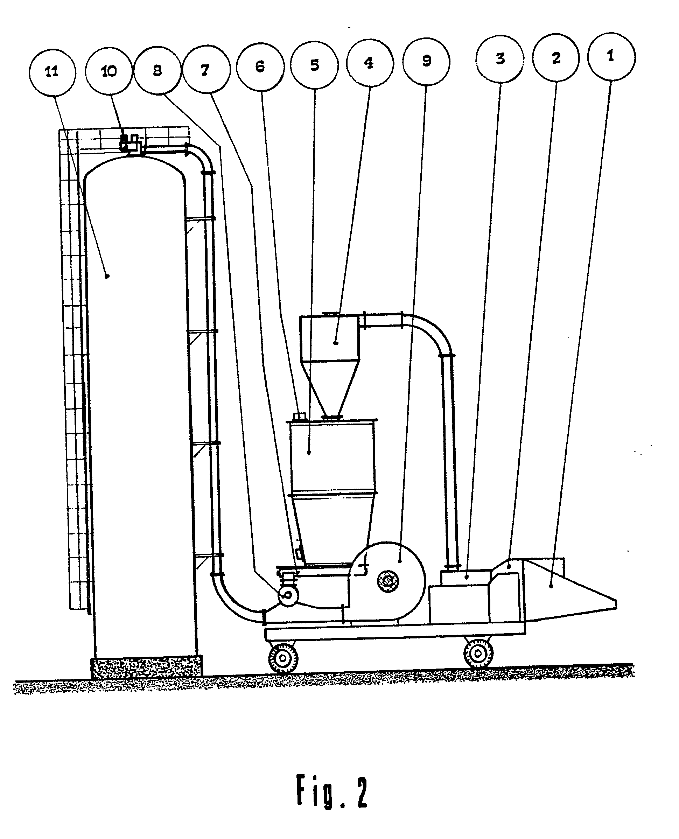

Figure 2 is a side view in the direction A.

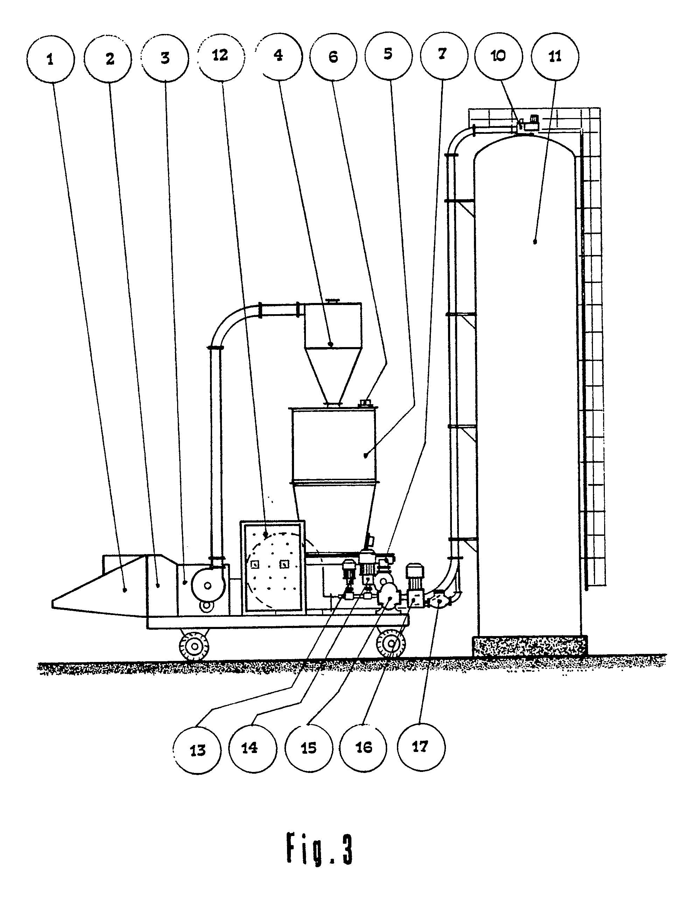

Figure 3 is a side view in the direction B.

The figures show diagrammatically the following parts:

(1) a pneumatic or mechanical variable speed feeder;

(2) a knife shredder or equivalent;

(3) a breaking and grinding mill with mechanical or pneumatic conveying systems; the mill is also provided with a fan for raising the ground straw to subsequent apparatus, including silos;

(4) a decantation cyclone for the ground product;

(5) a container for the ground product, comprising internally a breakdown wheel or equivalent system;

(6) rotary or equivalent level indicators;

(7) a metering extractor for the straw, of screw type or an equivalent mechanical or pneumatic system;

(8) a fluidising rotary valve or equivalent;

(9) an electric motor-driven fan for mixing the product and conveying it to the subsequent apparatus;

(10) a turboatomiser or equivalent mechanical system for distributing liquids and ground straw;

(11) a fermentation silo;

(12) a control panel;

(13) a liquid microdispenser;

(14) a liquid dispenser;

(15) a liquid pump;

(16) a continuous mixer;

(17) a flow meter.

The apparatus enables any fibrous material of vegetable origin to be processed and subsequently fermented. Again, reference will be made for simplicity only to the processing of straw, but this term is intended to include any waste vegetable material. The following operations are carried out in practice:

a) the straw is loaded into the variable speed feeder (1). The feed speed is adjusted either manually or automatically, according to the rate at which the processed mass is loaded into the silo.

b) The straw undergoes a first physical change in the shredder (2) and mill (3), where it is reduced to a mass of fragments having a length of between 0.1 and 7 mm.

c) The ground straw is fed to storage. The mass is conveyed from the mill into the decanting cyclone (4), from which it passes by gravity into the collection buffer tank (5) in which the quantity of straw present is controlled by the level indicators (6), preferably of rotary type, which when the tank is too full transmit a signal to the mill to temporarily halt it, and when the tank is too empty transmit a signal to the metering extractor unit (7), in order to halt it temporarily.

d) The ground straw is metered by means of the extractor (7) and conveyed by an electric motor-driven fan (9), which is fitted with a straw inlet valve (8), so as to allow automatic control of the ratio of weight of cellulose material to the air volume, and thus to allow the formation of a homogeneous constant stream of conveyed material.

An aqueous solution of $KMnO_4$, and an aqueous solution of $CUSO_4$, $FeSO_4$, molasses and urea are prepared separately, and a water supply is made available.

The $KMnO_4$ solution is fed by the microdispenser (13), the solution of the remaining components is fed by the dispenser (14), and the water is fed by the pump (15), all suitably controlled so that in the continuous mixer (16) a homogeneous solution forms in which the components are present within the previously fixed limits.

The solution according to the invention is pumped from the mixer (16), through the flow meter (17) and to the turboatomiser (10), by means of the pumping assembly (13), (14), (15).

e) The straw is mixed with the solution. The continuous stream of ground straw reaches the turboatomiser (10) simultaneously with the treatment solution. The turboatomiser (10) also comprises a rotating cap which facilitates the uniform mixing of the solid with the liquid in the required proportions. This system also ensures that the impregnated mass is uniformly distributed and collected in the underlying silo.

f) The mass contained in the silo is fermented. The progress of the fermentation in the silo can be

# 0 076 964

followed either by temperature probes which measure the temperature inside the mass at various times and thus enable the temperature-time curve to be determined, or by withdrawing samples of the fermenting mass at determined times and mesauring the significant parameters of Table 1. When fermentation has finished, the temperature of the product is about 40°C.

It has also been found that if the head requirements allow it, the apparatus elements (4), (5), (6), (7), (8), (9) are no longer essential to ensure the conveying of the ground straw mass to the silo (11), and instead said mass can be fed into the silo through the turboatomiser (10) directly by means of the fan present in the mill (3).

The premixed treatment solution reaches the turboatomiser (10) as already stated.

In a further simplification of the process, in particular for batch operation, the continuous mixer (16) can be dispensed with, and the individual solutions fed directly to the turboatomiser (10).

A practical example is described hereinafter in order to make the process according to the present invention more easily reproducible, but without limiting the scope of its application in any way.

Example

Having switched on the various controls of the electrical panel (12), the machine is put into the automatic operating mode.

The bales of straw are loaded onto the feeder (1) and fed into the mill (3) by way of the shredder (2).

When the level indicator (6) feeds an input signal to the electrical panel, this has the effect of starting the extraction mechanism (7), the fluidising rotary valve (8), the electric motor-driven conveying fan (9), and the pump assembly (13), (14), (15), which for every 100 kg of straw feeds 230 kg of water containing 3.15 kg of molasses, 2.60 kg of urea, 0.05 kg of $FeSO_4$, 0.01 kg of $CuSO_4$, 0.270 kg of dicalcium phosphate and 0.003 kg of $KMnO_4$ to the turboatomiser (10) by way of the continuous mixer (16).

The turboatomiser (10) provides for uniformly distributing the mixture (ground straw-fermentation solution), which is left to ferment for thirteen days. After this time, a sample is withdrawn, and on analysis gives the following results, expressed with reference to the dry substance:

| | |
|---|---|
| — total nitrogenated substances | 7.6% |
| — lipids | 0.5% |
| — fibre | 38.4% |
| — ash | 8.2% |
| — extractable non-nitrogenated | 45.3% |
| — acetic acid | 4.10% |
| — butyric acid | 0.66% |
| — propionic acid | 0.55% |
| total fatty acids | 5.31% |
| pH | 6.9 |

## Claims

1. A livestock feed of high nutrient value from waste vegetable materials of high cellulose and lignin content, characterized in that it is obtained by the process comrpising the following steps:

a) the vegetable material is ground into pieces having a length not exceeding 7 mm;

b) the ground material is impregnated with a solution having the following composition per 100 kg of treated material:

| | |
|---|---|
| water | 200—300 litres |
| $KMnO_4$ | 0.25—0.35 g |
| $FeSO_4 . 7H_2O$ | 40—60 g |
| $CuSO_4 . 5H_2O$ | 8—12 g |
| dicalcium phosphate | 250—500 g |
| molasses | 1—5 kg |
| urea | 1.5—3.5 kg |

5

**0 076 964**

c) the mass is left to ferment for 2—25 days, preferably for 12—20 days.

2. A livestock feed according to claim 1, characterized in that the impregnation solution is used at 50°C, with the minimum stated quantity of urea and the maximum stated quantity of phosphate, and in that the fermentation storage is about two days.

3. An apparatus for producing a livestock feed according to claim 1, which comprises essentially a feeder (1) for the vegetable material, a shredder (2) for this latter, a mill (3) provided with a fan for raising the material, a turboatomiser (10) for uniformly impregnating the vegetable material with the fermentation solution, a pump-dispenser unit (13), (14), (15) for the solution, and a silo (11) for the fermentation.

4. An apparatus as claimed in claim 3, wherein the following components are also present: a cyclone (4) for decanting the ground material, a container (5) for the ground material, level indicators (6), a metering extractor for the vegetable material (7), a rotary valve (8), and an electric motor-driven fan (9).

**Revendications**

1. Aliment pour bétail de haute valeur nutritive, tiré de matières végéales de rebut à forte teneur en cellulose et en lignine, caractérisé en ce qu'il est obtenu par le procédé comprenant les phases suivantes:

a) la matière végétale est broyée en fragments ayant une longueur qui ne dépasse pas 7 mm;

b) la matière broyée est imprégnée d'une solution ayant la composition suivante, pour 100 kg de matière traitée:

| | |
|---|---|
| Eau | 200—300 litres |
| $KMnO_4$ | 0,25—0,35 g |
| $FeSO_4 . 7H_2O$ | 40—60 g |
| $CuSO_4 . 5H_2O$ | 8—12 g |
| Phosphate dicalcique | 250—500 g |
| Mélasse | 1—5 kg |
| Urée | 1,5—3,5 kg; |

c) la masse est mise à fermenter pendant 2 à 25 jours, de préférence pendant 12 à 20 jours.

2. Aliment pour bétail selon la revendication 1, caractérisé en ce que la solution d'imprégnation est utilisée à 50°C, avec la quantité minimale spécifiée d'urée et la quantité maximale spécifiée de phosphate, et en ce qu'on laisse fermenter pendant deux jours environ.

3. Dispositif pour la production d'un aliment pour bétail selon la revendication 1, comprenant essentiellement un chargeur (1) pour la matière végétale, une machine à déchirer le fourrage (2) pour cette matière, un broyeur (3) muni d'un ventilateur pour élever la matière, un atomiseur à turbine (10) pour imprégner uniformément la matière végétale avec la solution de fermentation, un groupe pompe-dispensateur (13), (14), (15) pour la solution et un silo (11) pour la fermentation.

4. Dispositif selon la revendication 3, dans lequel il est aussi prévu les éléments suivants: un cyclone (4) pour séparer la matière broyée, un réservoir (5) pour la matière broyée, des indicateurs de niveau (6), un extracteur-doseur (7) pour la matière végétale, une vanne rotative (8) et un ventilateur à moteur électrique (9).

**Patentansprüche**

1. Tierfutter mit hohem Nährwert aus pflanzlichen Abfallmaterialien mit hohem Zellulose- und Ligningehalt, dadurch gekennzeichnet, daß es nach dem Verfahren erhalten wird, welches die folgenden Schritte umfaßt:

a) das Pflanzenmaterial wird zu Stücken mit einer 7 mm nicht übersteigenden Länge gemahlen,

b) das gemahlene Material wird mit einer Lösung mit folgender Zusammensetzung pro 100 kg behandeltem Material imprägniert:

| | |
|---|---|
| Wasser | 200—300 l |
| $KMnO_4$ | 0,25—0,35 g |
| $FeSO_4 . 7H_2O$ | 40—60 g |
| $CuSO_4 . 5H_2O$ | 8—12 g |

6

| Dikalziumphosphat | 250—500 g |
| Melasse | 1—5 kg |
| Harnstoff | 1,5—3,5 kg |

c) die Masse wird 2 bis 25 Tage, vorzugsweise 12 bis 20 Tage, fermentieren gelassen.

2. Tierfutter gemäß Anspruch 1, dadurch gekennzeichnet, daß die Imprägnierlösung bei 50°C mit der minimal angegebenen Quantität Harnstoff und der maximal angegebenen Quantität Phosphat verwendet wrid und daß die Fermentationslagerung etwa 2 Tage beträgt.

3. Vorrichtung zum Herstellen eines Tierfutters gemäß Anspruch 1, welches im wesentlichen aus einer Zufuhreinrichtung (1) für das Pflanzenmaterial, einem Zerkleinerungsgerät (2) für das letztere, einer Mühle (3), die mit einem Gebläse zum Heben des Materials ausgestattet ist, einem Turbozerkleinerer (10) zum gleichmäßigen Imprägnieren des Pflanzenmaterials mit Fermentationslösung, einer Pumpen-Verteilereinheit (13), (14), (15) für die Lösung und einem Silo (11) für die Fermentation besteht.

4. Vorrichtung, wie in Anspruch 3 beansprucht, worin auch die folgenden Bestandteile vorhanden sind: ein Zyklon (4) zum Abtrennen des gemahlenen Materials, ein Behälter (5) für das gemahlene Material, Niveauindikatoren (6), ein Meßextraktor für das Pflanzenmaterial (7), ein Rotationsventil (8) und ein von einem Elektromotor angetriebenes Gebläse (9).

B

6 17 16 15 14 13 4 12

11 10 8 7 5 9 3 2 1

A

Fig. 1

Fig. 2

Fig.3